# EUROPEAN PATENT APPLICATION

(11) **EP 2 666 515 A1**
(43) Date of publication of application: **27.11.2013**
(21) Application number: 12169407.9
(22) Date of filing: 24.05.2012
(51) Int. Cl.: A61N 2/02

(54) **Transcranial magnetic stimulation for small animals**

(71) Applicant: Universiteit Antwerpen, 2000 Antwerpen (BE)
(72) Inventor: Staelens, Steven, 9820 Merelbeke (BE); Wyckhuys, Tine, 9000 Gent (BE)
(74) Representative: Hertoghe, Kris Angèle Louisa

(57) **Abstract**

The present invention provides a positioning device (1) for positioning a transcranial magnetic stimulation coil (15). The positioning device (1) comprises a support element (4) adapted for implantation at a cranial stimulation site of an animal, and at least one protrusion (6) connected to the support element (4) for extending transdermally when the support element (4) is implanted. The at least one protrusion (6) is adapted for mechanically connecting with the transcranial magnetic stimulation coil (15) for administering transcranial magnetic stimulation at said cranial stimulation site of said animal. In a further aspect, the invention provides in a kit of parts comprising such positioning device (1) and a transcranial magnetic stimulation coil (15). Such kit of parts may further comprise a stimulation pulse source (21) for supplying a pulsed electrical signal to the transcranial magnetic stimulation coil (15).

## Description

### Field of the invention

The present invention relates to the field of magnetic neurostimulation. More specifically it relates to transcranial magnetic stimulation of small animals, for example for preclinical research.

### Background of the invention

Transcranial magnetic stimulation (TMS) is a non-invasive technique which makes use of a rapidly varying magnetic field applied to a position on the head in order to induce electrical currents in the brain. The induced currents may cause an activation or inhibition of specific areas of the brain. TMS may be applied for diagnostic purposes, e.g. for functional mapping of the human cortex or for diagnosis of demyelinating diseases such as multiple sclerosis, by measuring the latency time between activation of the motor cortex by single pulse TMS and the response in the muscle detected by electromyographic measurements.

More recently, repetitive TMS (rTMS) has been used in the treatment of diverse chronic neurological and psychiatric disorders, such as addiction, migraine, tinnitus and epilepsy. Furthermore, rTMS has been FDA-approved since 2008 for the treatment of depression. Development of non-pharmaceutical and non-toxic treatment for neurological disorders is a growing area of research, as in 20-30% of the patients having such brain disorders the current pharmaceutical treatments may not prove effective and in an additional 20-30% of the patients such pharmaceutical therapy may be suboptimal or may have significant side-effects.

Although rTMS is a non-invasive and easily applicable form of therapy which has yielded promising clinical results, the therapeutic potential of rTMS remains underused due to limited knowledge about the optimal stimulation parameters, optimal stimulation target in the brain, optimal TMS coil design and generally insufficient knowledge of the working principles and effects of rTMS.

The choice of currently used stimulation parameters in human applications is up until now primarily based on trial and error. Stimulation is mostly applied at frequencies between 1 Hz and 25 Hz, and this has remained unchanged since the initial studies about the use of TMS. However, a large margin for improvement may exist for the stimulation frequency. For example, for another form of neural stimulation, deep brain stimulation (DBS), it has been found that stimulation frequencies above 100 Hz, or random frequency stimulation, induce a stronger effect than the conventionally applied low frequencies. Furthermore, the choice of dimensions and shape of the TMS coil for the treatment of patients is primarily based on empirical findings. Figure-eight shaped or circularly shaped coils are typically preferred. Nevertheless, it is known that the number of windings, the number of loops, the wire gauge, the material composition, the conicity of the coils, etc. may influence to a large extent the focality of the induced electrical field and hence neurophysiological response.

Since human research into optimization and working principles is limited due to ethical considerations and the necessity of large and homogenous groups of patients, preclinical research in which TMS is applied to experimental animals may be crucial in gaining a more thorough understanding of TMS. However, the currently commercially available TMS stimulators are intended for use in human patients or test subjects and the focus of the field of such stimulators may therefore be inadequate for accurately stimulating targeted brain areas in these small animals. Such stimulators may furthermore only offer limited flexibility regarding the choice of stimulation parameters such as pulse width, frequency, stimulation pattern, intensity and total stimulation time, e.g. may only offer a limited range of such parameters only consistent with currently known clinical paradigms in humans, but not suitable for extensive experimentation and protocol optimization.

In D. Liebetanz et al, "Safety aspects of chronic low-frequency transcranial magnetic stimulation based on localized proton magnetic resonance spectroscopy and histology of the rat brain," Journal of Psychiatric Research, volume 37 (2003), pages 277-286, a transcranial magnetic stimulation coil and stimulator specifically adapted for small mammals are disclosed. It is however an important disadvantage of the system there disclosed that positioning the coil on the animal head in a reproducible way may be difficult. This could lead to variable results due to the absence of focus on the brain target region in small mammals hence limiting the translational validity towards human applicability. Also in such system it may be impossible to apply TMS to small mammals that are awake and freely moving, which implies that no behavioural or unsedated imaging monitoring can be performed.

### Summary of the invention

It is an object of embodiments of the present invention to provide good and accurate transcranial magnetic stimulation to small animals.

The above objective is accomplished by a method and device according to the present invention.

In a first aspect, the present invention provides a positioning device for positioning a transcranial magnetic stimulation coil. The positioning device comprises a support element adapted for implantation at a cranial stimulation site of an animal, in particular e.g. a small animal such as a rodent, a rabbit or a monkey, and at least one protrusion arranged on the support element for extending transdermally when the support element is implanted. The at least one protrusion is adapted for mechanically connecting with a transcranial magnetic stimulation coil for administering transcranial magnetic stimulation at this cranial stimulation site of the animal.

In a positioning device according to embodiments of the present invention, the support element may be adapted, e.g. in shape, size and/or material, for bone-anchoring to the skull bone of said animal.

In a positioning device according to embodiments of the present invention, the support element may be composed of a biocompatible material.

In a positioning device according to embodiments of the present invention, the support element may be a flat body.

In a positioning device according to embodiments of the present invention, the support element may be flexible.

In a positioning device according to embodiments of the present invention, the support element may have at least one hole or groove for promoting tissue growth therein.

In a positioning device according to embodiments of the present invention, the support element may have at least one hole to accurately position the support element at the transcranial stimulation site. The at least one hole to accurately position the support element at the transcranial stimulation site may be the same as the at least one hole for promoting tissue growth therein. Such hole may first be used for accurately positioning the support element, and thereafter tissue growth may be promoted therein.

In a positioning device according to embodiments of the present invention, the at least one protrusion may comprise at least one elongate guide element adapted in shape to receive a through-hole of the transcranial magnetic stimulation coil.

In a positioning device according to embodiments of the present invention, the at least one protrusion may comprise at least two protrusions. In particular embodiments, the at least two protrusions may be at least two elongate guide elements for receiving at least two corresponding through-holes of a correspondingly shaped transcranial magnetic stimulation coil. Depending on the number of protrusions, the coil may for example be figure-eight shaped, clover shaped, or may have any other suitable coil design.

In a positioning device according to embodiments of the present invention, the at least one elongate guide element may comprise four elongate guide elements for receiving four corresponding through-holes of a clover-shaped transcranial magnetic stimulation coil.

A positioning device according to embodiments of the present invention may furthermore comprise a locking means for fixing the transcranial magnetic stimulation coil into place when connected to the at least one protrusion.

In a positioning device according to embodiments of the present invention, the support element may be adapted for implantation at a cranial stimulation site of a rodent. Such adaptation may for example consist in an adaptation in size and/or shape and/or material properties of the support element.

In a second aspect, the present invention provides in a kit of parts comprising a positioning device according to embodiments of the first aspect of the present invention and a transcranial magnetic stimulation coil adapted in shape for mechanically connecting with the at least one protrusion.

In a kit of parts according to embodiments of the present invention, the transcranial magnetic stimulation coil may comprise at least one cooling element.

A kit of parts according to embodiments of the present invention may furthermore comprise a stimulation pulse source for supplying a pulsed electrical signal to the transcranial magnetic stimulation coil.

A kit of parts according to embodiments of the present invention may furthermore comprise a feedback loop to record biological signals of the animal.

A kit of parts according to embodiments of the present invention may furthermore comprise a biocompatible adhesive, e.g. a glue or cement, for anchoring the support element to the skull bone of the animal.

It is an advantage of embodiments of the present invention that transcranial magnetic stimulation may be applied in an accurately defined target region of the animal brain. Whole brain stimulation may be avoided.

It is an advantage of embodiments of the present invention that transcranial magnetic stimulation may be applied to small animals while behavioural testing is performed on the animal subject.

It is an advantage of embodiments of the present invention that transcranial magnetic stimulation may be applied to small animals while being awake and freely moving.

It is an advantage of embodiments of the present invention that transcranial magnetic stimulation may be applied to small animals while performing a functional imaging technique, such as positron emission tomography, on the animal.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

FIG. 1 shows a positioning device according to a first embodiment of the present invention.
FIG. 2 shows a positioning device according to a second embodiment of the present invention.
FIG. 3 shows the positioning device of FIG. 2 with a corresponding magnetic stimulation coil.
FIG. 4 illustrates the implantation of a positioning device according to embodiments of the present invention in a small experimental animal.
FIG. 5 shows a small experimental animal having a positioning device according to embodiments of the present invention implanted.
FIG. 6 illustrates the application of TMS to a small experimental animal with the aid of a positioning device according to embodiments of the present invention.
FIG. 7 shows a first exemplary experimental setup with a positioning device according to embodiments of the present invention.
FIG. 8 shows a second exemplary experimental setup with a positioning device according to embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a positioning device for positioning a transcranial magnetic stimulation coil. This positioning device comprises a support element adapted for implantation at a cranial stimulation site of an animal, e.g. a small experimental model animal. The positioning device also comprises at least one protrusion arranged on the support element, e.g. at least one elongate guide element connected to the support element, for extending transdermally when the support element is implanted on the skull of the animal. This at least one protrusion is adapted for mechanically connecting with a transcranial magnetic stimulation coil, e.g. for receiving a stimulation coil sliding over it or clicking onto it, for administering transcranial magnetic stimulation at the cranial stimulation site of the animal.

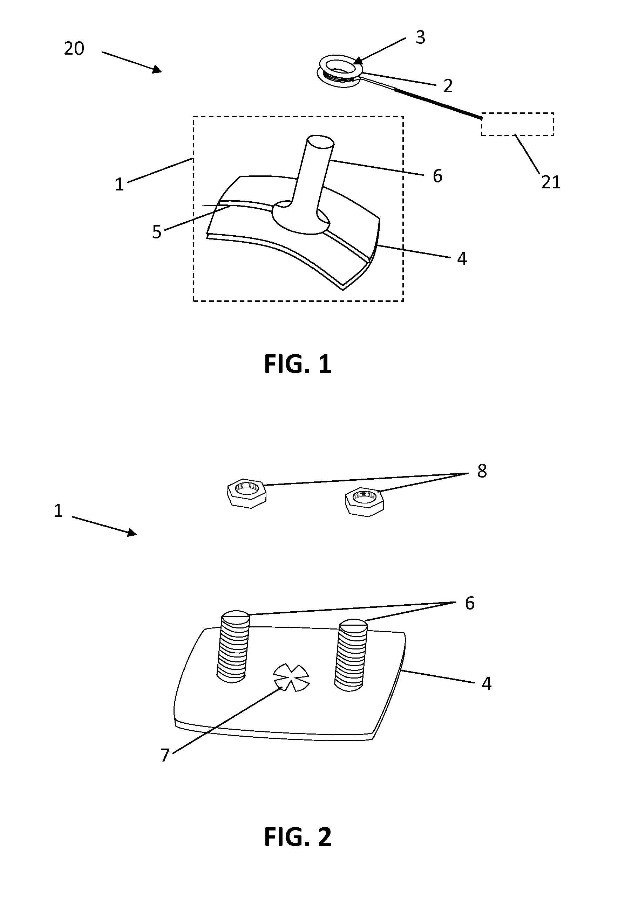

Referring to FIG. 1, a positioning device 1 for positioning a transcranial magnetic stimulation coil 2 according to a first embodiment of the present invention is shown. The positioning device 1 comprises a support element 4, which is adapted for implantation at a cranial stimulation site of an animal, e.g. for implantation on the skull bone at this cranial stimulation site of the animal. For example, such animal may be a vertebrate, such as a mammal, e.g. a cat or other feline, a rabbit or a monkey. Particularly, the support element 4 may be adapted for implantation at a cranial stimulation site of a rodent, such as a rat or a mouse. Particularly, the support element 4 may be adapted in shape to fit onto the cranial bone at the cranial stimulation site. For example, the support element 4 may be adapted for bone-anchoring to the skull bone of the animal, e.g. such that the support element 4 may be fixed to the cranial bone by suitable fixing means, such as a biocompatible glue, e.g. a one-component or two-component biocompatible glue, a dental cement or at least one screw. Such fixation of the support element 4 to the cranial bone may have the advantage of providing sufficient structural support for retaining the magnetic stimulation coil in a fixed position and orientation with respect to the brain structures below the cranial stimulation site. For example, the support element 4 may be relatively thin, e.g. may have a thickness which is substantially smaller than its diameter, for example a thickness less than 25% of its diameter, e.g. less than 10%, less than 5% or even less than 1% of its diameter. It is to be noted that herein the diameter of the support element 4 refers to the largest distance in a straight line that can be formed between two points on the surface of the support element 4. The support element 4 may be, but does not need to be, of uniform thickness. The support element may furthermore be flat, e.g. be substantially planar in an unstressed state. As examples, without being limiting, the support element 4 may have a flat circular, oval, elliptic, rectangular or square shape. For example, the support element 4 may be a circular body having a diameter between 5 mm and 5 cm, for example a diameter of 1 cm, and a thickness between 0.5 mm and 3 mm, e.g. a thickness of 1 mm. Furthermore, the support element 4 may be flexible. It is an advantage of such flexible support element 4 that the support element 4 can adapt to a contour of the skull at the cranial stimulation site when implanted. It will be apparent to the person skilled in the art that the spatial dimensions of the support element 4 may be determined by the shape and dimensions of the skull of the animal for which the positioning device 1 is intended. For example, for a positioning device 1 according to embodiments of the present invention, which is adapted for implantation in a rat, the diameter, e.g. the width, of the support element may be less than or equal to 15 mm.

The support element 4 according to embodiments of the present invention may be composed of a biocompatible material, for example a biocompatible metal or metal alloy, such as implant-grade stainless steels and related alloys, titanium and titanium alloys or even high-karat gold, a biocompatible medical-grade synthetic material, such as moulded silicones, Teflon (PTFE), nylons or polyurethane, or another biocompatible material, such as glass. On the other hand, materials that degrade under the conditions in the mammal body are to be avoided, such as organic materials and degradable polymers. While the support element 4 may be entirely composed of such biocompatible material, the support element 4 may also consist of or comprise a non-biocompatible material having a biocompatible coating applied thereto or a non-biocompatible material which is encapsulated in a biocompatible material.

The support element 4 may furthermore comprise additional features for improving the cranial fixation of the support element 4 when implanted. For example, the support element 4 may have at least one hole 7 (as illustrated in the embodiments of FIG. 2) or groove 5 (illustrated in the embodiment of FIG. 1). When the support element 4 has such grooves 5 or holes 7 provided therein, a better fixation of the support element 4 may be achieved because, after implantation, tissue may grow into these grooves or through such holes. Furthermore, the support element 4 may have at least one hole 7 to accurately position the support element 4 at the cranial stimulation site, e.g. a hole 7 at a position which may indicate the surface point on the skull below which the focus spot of the transcranial magnetic stimulation coil 2 when connected to the positioning device 1 will be situated, see for example the cross-hair shaped hole 7 in FIG. 2. Such accurate positioning may comprise positioning of the positioning device 1 on the animal skull such that a desired brain structure is accurately targeted by the stimulation coil 2 when connected to the positioning device 1. This brain structure may be mapped by stereotactic coordinates. For example, a brain structure may be targeted by implantation at a cranial stimulation site which is found at coordinates relative to a cranial landmark point, such as the bregma. The hole 7 may therefore assist in accurate positioning of the positioning device 1 relative to such cranial landmark, e.g. the bregma.

Furthermore, a bio-compatible adhesive, e.g. a surgical adhesive for internal use, may be applied to the support element 4 in order to improve fixation when implanted, for example for fixation of the support element 4 onto the skull bone. A multi-component adhesive may be used, in which, for example, one component may be pre-applied to the support element 4 and the other component may be provided for application on the cranial stimulation site. A one-component adhesive may also be used, and may be, for example, supplied separately for application to the support element 4 and/or cranial stimulation site during implantation. Alternatively, a one-component adhesive may be pre-applied to the support element 4.

The positioning device 1 further comprises at least one protrusion 6 connected to the support element 4. This at least one protrusion 6 is adapted for protruding transdermally when the support element 4 is subcutaneously implanted. For example, the length of this protrusion 6 may be substantially larger than the dermal thickness at the cranial stimulation site of the animal, such that, when the support element 4 is implanted at this site, tissue may regrow to cover the support element 4 while leaving the at least one elongate guide element 6 at least partially exposed. The at least one protrusion 6 and the support element 4 may be integrally formed by moulding, extrusion or similar manufacturing processes. Alternatively, the at least one protrusion 6 may comprise at least one elongate guide element which is securely attached to the support element 4, e.g. glued, welded, soldered, riveted or screwed thereon.

The at least one protrusion 6 is furthermore adapted for mechanically connecting with a transcranial magnetic stimulation coil 2 for administering transcranial magnetic stimulation at the cranial stimulation site of the animal. For example, in a first embodiment of the present invention, e.g. as shown in FIG. 1, the at least one protrusion 6 may comprise at least one elongate guide element 6, for example one elongate guide element 6 adapted in shape to receive a central through-hole 3 of a transcranial magnetic stimulation coil 2, e.g. the elongate guide element 6 may fit into such a central through-hole 3 in order to accurately position the magnetic stimulation coil at the cranial stimulation site and to hold this coil firmly into place while administering transcranial magnetic stimulation.

The at least one protrusion 6 may furthermore be adapted for adjusting the electric and/or magnetic field induced by the TMS coil 2, e.g. to intensify such fields, to improve the focal properties of such fields or to improve the depth penetration of these fields. For example, the at least one protrusion 6 may be composed of or may comprise a material having suitable magnetic properties, e.g. a ferromagnetic material.

Furthermore, the positioning device 1 may comprise a shielding element, arranged such that the induced magnetic and/or electric field induced by the TMS coil 2 is selectively shielded in order to improve the focality of the transcranial magnetic stimulation. For example, such shielding element, e.g. a shielding plate, may be arranged between the TMS coil 2 when connected to the at least one protrusion 6 and the support element 4.

It will be apparent to the person skilled in the art that the spatial dimensions of such at least one protrusion 6 may be determined by the shape and dimensions of the skull of the animal on one hand and the dimensions of the transcranial magnetic stimulation coil 2 on the other hand. For example, for a positioning device 1 according to embodiments of the present invention, which is adapted for implantation in a rat, the protrusion 6 may comprise an elongate guide element which may be, for example, cylindrically shaped with a thickness in the range of 1 mm to 5 mm, for example a thickness of 2 mm, and a height in the range of 10 mm to 15 mm, for example 12 mm. Furthermore, the positioning device 1 may be adapted for receiving a figure-eight transcranial magnetic stimulation coil adapted for transcranial magnetic stimulation of a rat, e.g. the at least one protrusion 6 may comprise two elongate guide elements spaced, for example, at a distance of about 10 mm, e.g. corresponding to the distance between the through holes of the coil windings.

Referring to FIG. 2, in a second embodiment of the present invention, the at least one protrusion 6 may comprise at least two elongate guide elements, e.g. may comprise two elongate guide elements, for receiving corresponding through-holes 3 of a suitable shaped transcranial magnetic stimulation coil 15, e.g. for receiving two corresponding through-holes 3 of a figure-eight shaped transcranial magnetic stimulation coil 15. For example, these two elongate guide elements 6 may have identical shape and dimensions and may be oriented parallel to one another, for example perpendicular to the support element 4.

Similarly, the at least one elongate guide element may comprises four elongate guide elements for receiving four corresponding through-holes 3 of a clover-shaped transcranial magnetic stimulation coil 15. Furthermore, while the at least one protrusion 6 may be arranged substantially perpendicular to the support element 4, this at least one protrusion 6 may also be arranged at an angle with respect to the support element 4, for example, at an angle of 60°, at an angle of 45°, or at an angle of 30°.

Examples are given hereinabove with two or four protrusions 6, the invention, however, not being limited thereto. Any other suitable number of protrusions may also be used and also forms part of the present invention, e.g. three protrusions. The number of protrusions may typically, but does not necessarily need to, correspond to the number of mechanical connection locations of the stimulation coil. There may be more or fewer protrusions than mechanical connection locations of the stimulation coils, e.g. centre holes in windings of the stimulation coil adapted in shape for receiving one of the protrusions, provided the location of the protrusions and the shape of the coil are adapted to one another for allowing mechanical connection of the coil onto the protrusions. As an example, three protrusions may be provided, while the coil is figure-eight shaped with only two holes for receiving protrusions. In such case, the third protrusion should be provided on the support element at such location that the figure-eight shaped coil can still be mechanically connected to the support element. It is an advantage of such support element that it allows different types of stimulation coils to be connected, e.g. both stimulation coils with two receiving holes for receiving protrusions of the support element, and stimulation coils with three such receiving holes.

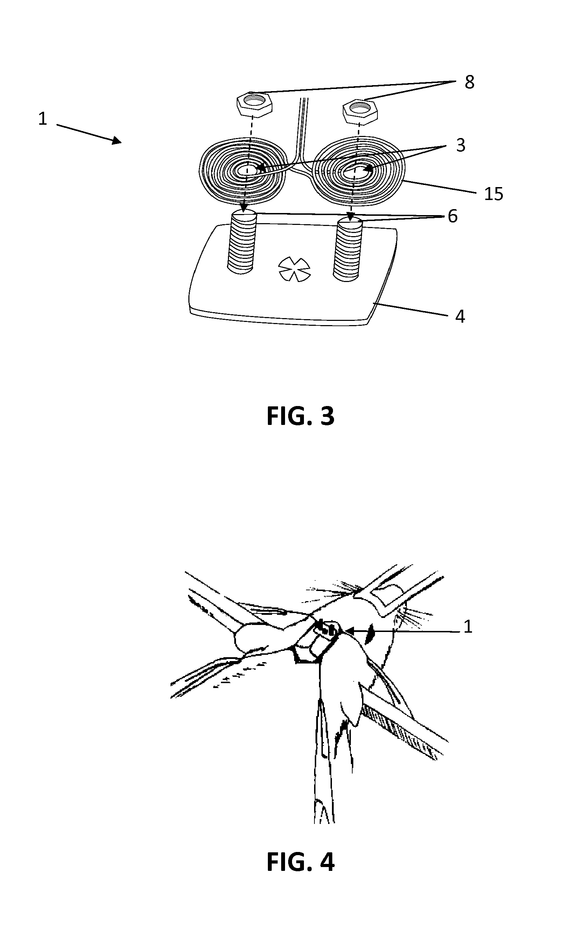

The positioning device 1 may furthermore comprise a locking means 8 for fixing the transcranial magnetic stimulation coil 2 into place when mechanically connected with the at least one elongate guide element 6. For example, the locking means 8 may comprise at least one fastener with a threaded hole, e.g. a nut, which fits onto a corresponding thread provided on the at least one elongate guide element 6. The thread may be, but does not need to be, provided over substantially the complete surface of the at least one elongate guide element 6. For example, FIG. 2 shows two elongate guide elements 6 provided with a thread, and two nuts 8 for fastening a figure-eight shaped transcranial magnetic stimulation coil 2 into place, such as further illustrated in FIG. 3.

Alternatively, the at least one protrusion 6 may comprise a resiliently deformable structure which may be introduced into a through hole of the transcranial magnetic stimulation coil 2 while being held in a deformed state, such as to fix the TMS coil 2 into place by releasing this resiliently deformable structure. For example, such resiliently deformable structure may be a homogenous mass of elastic material, which may be deformed, e.g. by elongation, such that the TMS coil 2 may slide over the protrusion 6 while subsequently being held firmly in place when releasing the elongating force. In another example, the at least one protrusion 6 may comprise a compressible chamber formed by an elastic wall, e.g. a closed, cylindrical gas-filled cavity formed in a rubber material, such that the protrusion 5 may be squeezed into a through-hole of the TMS coil 2. In yet another example, such resiliently deformable structure may comprise an annular structure of resilient material, such that the through-hole of the TMS coil 2 may fit over this annular structure, in a manner similar to the locking mechanism known for holding compact discs in CD cases.

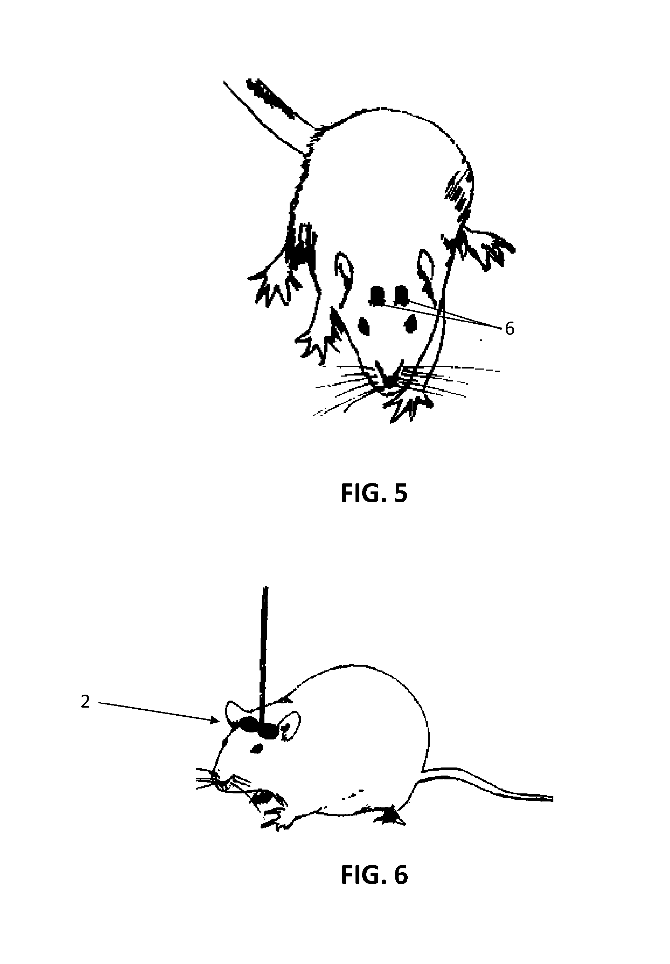

An exemplary procedure for implanting the positioning device 1 according to embodiments of the present invention is shown in FIG. 4. An incision in the scalp is made to expose part of the skull, e.g. so as to reveal the skull seams, e.g. the intersections of the skull plates, and in particular for example the bregma or other reference structures, e.g. lambda. The positioning device 1 is fixed to the skull, e.g. by application of a bio-compatible adhesive, e.g. at a predetermined distance from the reference structure such as the bregma, so that stimulation patterns may be applied at a well-defined pre-determined target location of the brain. The position of the positioning device 1 may be accurately determined by using a stereotactic reference frame, such that the cranial stimulation site corresponds to a location on the skull for stimulating a predetermined targeted region of the brain. After insertion of the positioning device 1, the scalp may regrow to cover the support element 4, eventually leaving only the at least one elongate guide element 6 at least partially exposed, as illustrated in FIG. 5.

TMS may then be administered to the animal, while this animal is awake and may be allowed to move freely, e.g. as illustrated in FIG. 6, where a TMS coil 2 is connected to the at least one elongate guide element 6. It is an advantage of a positioning device 1 according to embodiments of the present invention that TMS can be administered at a well-defined cranial stimulation site while the animal is awake and freely moving. Therefore, the animal does not need to be anesthetised while TMS is administered, which otherwise would interfere with brain activity and therefore potentially disturb the experiment. Furthermore, the animal may perform behavioural tests while undergoing TMS. The power leads of the TMS coil may furthermore be supported by a wire guide system with a swivel to further improve free movement of the animal.

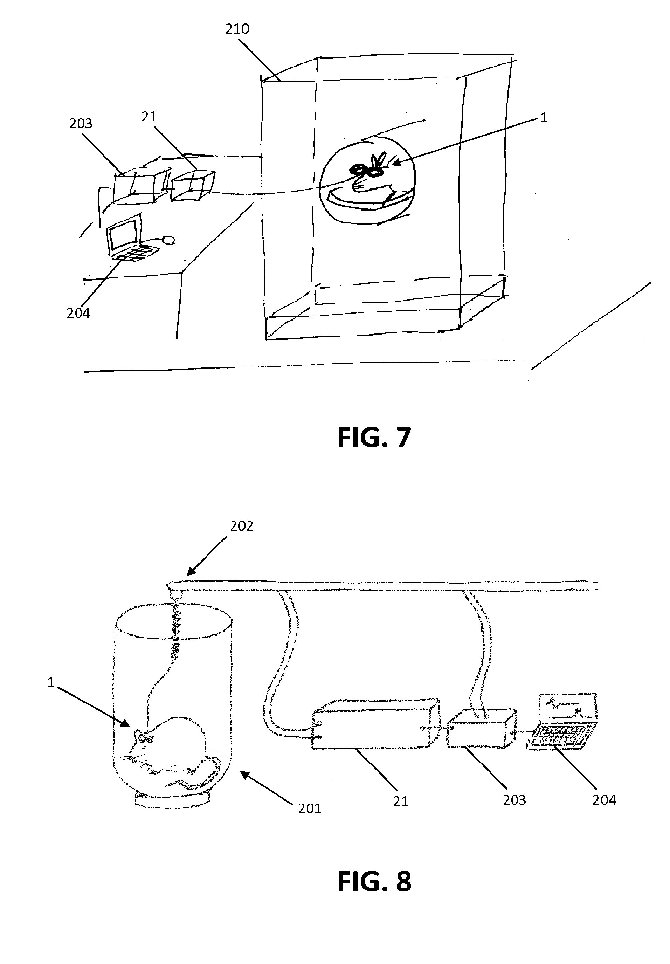

Referring to FIG. 8, an exemplary experimental setup is shown in which an animal is provided with a positioning device 1 according to embodiments of the present invention. In this exemplary experimental setup the animal may be subjected to behavioural testing, e.g. while allowed to move freely in a test area 201. A swivel system 202 or turnable cage may support electrical wires while allowing the animal to move freely. A stimulation pulse source 21, e.g. the stimulator, may provide electrical signals to the TMS coil attached to the positioning device 1 through such electrical wires. The stimulation pulse source 21 may be connected to a computing device, e.g. a computer 204, through an interface device 203. Furthermore, feedback signals may be received from the animal, for example through further electrical wires also guided by the swivel system 202 or turnable cave, and may be provided through the interface device 203 to the computer 204. For example, such feedback signals may comprise measurements of biological signals such as electroencephalography and/or electromyography data. The computer 204 may thus provide storage, visualisation and/or processing of such feedback signals, for example in isolation or in combination with the information regarding the supplied TMS pulses. Furthermore, the computer 204 may provide signals for providing a predetermined, e.g. pre-programmed, sequence of TMS pulses, or may provide signals for applying adaptive TMS in response to received feedback signals.

Referring to FIG. 7, another exemplary experimental setup is shown. In this setup, an animal provided with the positioning device 1 according to embodiments of the present invention, may be subjected to in-vivo imaging while subjected to TMS. The animal may be placed in an imaging apparatus 210 in order to obtain imaging data of the animal while receiving transcranial magnetic stimulation from a TMS coil connected to the positioning device 1. This experimental setup may furthermore comprise a stimulation pulse source 21 for providing pulsed electrical signals to the TMS coil. This stimulation pulse source 21 may be controlled by a computing device, e.g. computer 204, for example through an interface device 203. The imaging apparatus 210 may for example be a positron emission tomography unit (PET), a single-photon emission tomography unit (SPECT), a gamma camera, a magnetic resonance imaging unit (MRI) or a computed tomography unit (CT). Preferably, the imaging apparatus 210 is suitable for functional imaging of the animal brain. While the imaging apparatus 210 may operate independently from the stimulation pulse source 21, the imaging apparatus 210 may also be controlled by the computing device, e.g. computer 204.

In a second aspect, the present invention relates to a kit of parts 20, e.g. as shown in FIG. 1, comprising a positioning device 1 according to the first aspect of the present invention and a transcranial magnetic stimulation coil 2 adapted in shape for mechanically connecting with the at least one elongate guide element 6 of the positioning device 1. The transcranial magnetic stimulation coil 2 may furthermore be adapted for being supported by the support element 4 of the positioning device 1 when the positioning device 1 is implanted in a rodent, and the TMS coil 2 is mechanically connected to the at least one elongate guide element 6 of the positioning device 1. For example, the TMS coil 2 may be sufficiently small and light such that it can be supported by the rodent while freely moving without burdening the animal. For example, a TMS coil 2 suitable for stimulating a rat may be a figure-eight type coil weighing, for example, less than 10 gram, e.g. 7 gram, and having a thickness of less than 3 mm, for example 1.5 mm. Each of the two juxtaposed circular coil portions of such a figure-eight type TMS coil for rats may have a diameter of 2 cm. It will be apparent to the person skilled in the art that such weight and spatial dimensions are limited by the target animal, and, for example, for transcranial stimulation of a mouse a smaller TMS coil may be preferable than for stimulation of, for example, a rat.

The TMS coil 2 may for example comprise a circularly wound electrical conductor, e.g. a copper wire, or two asymmetrically wound coils, e.g. forming a figure-eight shaped TMS coil 15. For example, a circularly wound coil may have between 20 and 50 loops, e.g. 35 loops, of thin copper wire, for example having a cross-sectional diameter of 1 mm². The diameter of the TMS coil 2 may be less than 15 cm, and preferably less than 10 cm, for example about 5 cm, or even more preferred less than 5 cm, for example about 2 cm. In alternative embodiments, the TMS coil 2 may be of different design, e.g. may comprise a conically wound coil, or multiple leafs, for example a four-leaf coil.

The TMS coil 2, 15 may furthermore comprise at least one cooling element (not illustrated in the drawings), for example a cooling fan, piping for conducting a coolant, a heat exchange fin or a Peltier thermo-electric cooler. Furthermore, the coil may comprise a heat conductive casing for efficiently dispersing heat, e.g. a ceramic material having a high thermal conductivity.

In embodiments of the present invention, the kit of parts 20 may also comprise a stimulation pulse source 21 for supplying a pulsed electrical signal, e.g. a current or voltage, to the transcranial magnetic stimulation coil 2, 15. The stimulation pulse source 21 and transcranial magnetic stimulation coil 2, 15 may be adapted for providing a pulsed magnetic field having a maximum field strength in the range of 500 mT to 15 T, preferably in the range of 1 T to 8 T, for example about 7 T, or in the range of 1 T to 4 T, e.g. about 2 T. Such stimulation pulse source 21 may comprise a suitable electric amplifier, e.g. for providing an electric current to the TMS coil 2, 15, e.g. a monophasic or biphasic current, which may be controlled through a user interface, for example a user interface implemented in computer software. The stimulation pulse source 21 may be adapted for supplying a train of pulsed electrical signals, e.g. at a selectable frequency, for example in the range of 1 Hz to 200 Hz. Each pulse may have a time length of less than 10 ms, preferably less than 1 ms, for example a pulse width between 50 µs and 750 µs, e.g. 150 µs or preferably in the range of 300 µs to 600 µs, for example 450 µs. Each pulse may have a sinusoidal profile, or another predetermined profile, such as a triangular or block-wave profile. The stimulation pulse source 21 may also be adapted for enabling the selection of a waveform from a plurality of such pulse profiles. For example, referring to the exemplary experimental setup in FIG. 8, the stimulation pulse source 21 may be controlled by a computer 204 which may implement a user interface for selecting, visualizing and/or storing the stimulation parameters.

In embodiments of the present invention, the kit of parts 20 may also comprise a biocompatible adhesive for anchoring the support element 4 onto the skull bone of the animal.

## Claims

1. A positioning device (1) for positioning a transcranial magnetic stimulation coil (2, 15), the positioning device (1) comprising:
- a support element (4) adapted for implantation at a cranial stimulation site of an animal, and
- at least one protrusion (6) arranged on said support element (4) for extending transdermally when said support element (4) is implanted, said at least one protrusion (6) being adapted for mechanically connecting with a transcranial magnetic stimulation coil (2, 15) for administering transcranial magnetic stimulation at said cranial stimulation site of said animal.

2. The positioning device (1) according to claim 1, wherein said support element (4) is adapted for bone-anchoring to the skull bone of said animal.

3. The positioning device (1) according to any of the previous claims, wherein said support element (4) is composed of a biocompatible material.

4. The positioning device (1) according to any of the previous claims, wherein said support element (4) is a flat body.

5. The positioning device (1) according to any of the previous claims, wherein said support element (4) is flexible.

6. The positioning device (1) according to any of the previous claims, wherein said support element (4) has at least one hole (7) or groove (5) for promoting tissue growth therein.

7. The positioning device (1) according to any of the previous claims, wherein said support element (4) has at least one hole (7) to accurately position the support element (4) at the transcranial stimulation site.

8. The positioning device (1) according to any of the previous claims, wherein said at least one protrusion (6) comprises at least one elongate guide element adapted in shape to receive a through-hole (3) of said transcranial magnetic stimulation coil (2, 15).

9. The positioning device (1) according to claim 8, wherein said at least one elongate guide element (6) comprises at least two elongate guide elements for receiving at least two corresponding through-holes (3) of a correspondingly shaped transcranial magnetic stimulation coil (15).

10. The positioning device (1) according to claim 8, wherein said at least one elongate guide element (6) comprises four elongate guide elements for receiving four corresponding through-holes (3) of a clover-shaped transcranial magnetic stimulation coil (15).

11. The positioning device (1) according to any of the previous claims, furthermore comprising a locking means (8) for fixing said transcranial magnetic stimulation coil (2, 15) into place when connected to said at least one protrusion (6).

12. The positioning device (1) according to any of the previous claims, wherein said support element (4) is adapted for implantation at a cranial stimulation site of a rodent.

13. A kit of parts (20) comprising a positioning device (1) according to any of the previous claims and a transcranial magnetic stimulation coil (2, 15) adapted in shape for mechanically connecting with said at least one protrusion (6).

14. The kit of parts (20) according to claim 13, in which said transcranial magnetic stimulation coil (2, 15) comprises at least one cooling element.

15. The kit of parts (20) according to any of claim 13 or claim 14, furthermore comprising a stimulation pulse source (21) for supplying a pulsed electrical signal to said transcranial magnetic stimulation coil (2, 15).
